# EUROPEAN PATENT SPECIFICATION

(11) **EP 4 025 293 B1**
(45) Date of publication and mention of the grant of the patent: **25.02.2026**
(21) Application number: 20768709.6
(22) Date of filing: 03.09.2020
(51) Int. Cl.: A61N 1/04, A61B 5/259

(54) **ELECTRODE**
ELEKTRODE
ÉLECTRODE

(30) Priority: 04.09.2019 US 201962895714 P
(43) Date of publication of application: 13.07.2022
(73) Proprietor: Solventum Intellectual Properties Company, Maplewood, MN 55144 (US)
(72) Inventor: TRYPHONOPOULOS, John P., London, Ontario N5V 3R6 (CA); DERRY, Cameron E., London, Ontario N5V 3R6 (CA); NEUFELD, Howard P., London, Ontario N5V 3R6 (CA)
(74) Representative: Vossius & Partner Patentanwälte Rechtsanwälte mbB
(86) International application number: PCT/IB2020/058213
(87) International publication number: WO 2021/044339

(56) References cited:
- WO-A2-01/17423
- US-A- 3 901 218
- US-A1- 2017 340 232
- US-B1- 6 415 170

## Description

### Technical Field

The present disclosure relates to electrodes and methods of producing such electrodes.

### Background

Electrodes, such as electrocardiogram (ECG) electrodes, typically include a backing. The backing can be a thick foam backing that is punched to form a hole in the backing. Further, a separate covering or film is patched to the hole to create a cavity that contains a liquid gel. The covering adds additional material to a construction of such electrodes, and also requires a two-step operation with significant waste to create the cavity. Additionally, the step of punching the hole may be difficult to include in the fabrication process and may affect productivity rates. Further, the usage of thick backings may limit options for usage of alternative backings, such as thin films or non-woven films.

Some electrodes include a punched well without a foam backing. In such cases, there is usually a porous sponge or porous foam material that is placed over a sensor that is impregnated with the gel. However, using such porous sponges may limit a viscosity of the gel that is received within the punched well. More particularly, in such cases, the gel needs to be viscous enough so that the gel does not flow out of the porous sponge. Further, such electrodes additionally require a liner, that covers a skin-adhesive and a sponge face of the electrode, to have a dome shape so that the protruding gel/porous sponge combination can be accommodated and the electrode can lay flat.

US 3,901,218 A discloses a disposable medical electrode that includes a disk formed of a semi-rigid plastic material having a recessed embossment located centrally thereof. A metallic electrical contact is mounted on the disk centrally of the embossment and has portions thereof located on both sides of the disk. An absorbent pad positioned in the recessed embossment has absorbed therein an electrically conductive gel for forming an electrical contact between the patient on whom the electrode is positioned and the electrical contact thereby to permit electrical signals from the patient to be detected with the electrode by appropriate electrical equipment connected thereto. The disk has an adhesive applied thereto for firmly securing the electrode to the patient's skin.

US 2017/0340232 A1 discloses an electrode sensor. The electrode sensor can include a conductive sensor area that is at least partially covered by hydrogel. The hydrogel can be conductive and adhere to skin. A receptacle can form an open container surrounding the conductive sensor area and the hydroge

US 6 415 170 B1 shows a biomedical electrode comprising a backing material coated on one side with a pressure-sensitive adhesive and having a non-adhesive margin to facilitate the handling of the electrode; a connector stud located in the backing material, the stud having an electrode plate located on one side of the backing material for electrical connection to the skin of a patient and, on the other side of the backing material, a head portion to which an electrical connector can be attached; a strip of ionically-conductive adhesive extending, generally parallel to the non-adhesive margin, across the adhesive-coated side of the backing material and over the electrode plate of the stud; and a parallel strip of scrim material located at the interface of the ionically-conductive and pressure-sensitive adhesive; the strip of scrim material being displaced, relative to the electrode plate of the stud, towards the non-adhesive margin of the backing material and the edges of both strips nearest to the non-adhesive margin being positioned to one side of the electrode plate.

WO01/17423 A1 shows a biomedical electrode comprising: (a) a backing material coated on one side with a pressure-sensitive adhesive; (b) a connector stud located in the backing material, the stud having an electrode plate located on one side of the backing material for electrical connection to the skin of a patient and, on the other side of the backing material, a head portion to which an electrical connector can be attached; (c) a strip of ionically-conductive adhesive extending across the adhesive-coated side of the backing material and over the electrode plate of the stud; and (d) a parallel strip of scrim material located between the ionically-conductive and pressure-sensitive adhesives; the scrim material extending between the pressure-sensitive adhesive and the electrode plate of the stud, and having a width such that the electrode plate extends beyond at least one edge of the scrim material into contact with the pressuresensitive adhesive, and is adhered thereby to the backing material.

### Summary

The claimed invention concerns an electrode as defined by the appended independent claim 1. Further embodiments of the claimed invention are described in the appended dependent claims.

Aspects, embodiments, examples and methods described hereinafter are only of exemplary nature and are not restrictive for the invention which is defined by the appended claims.

The present disclosure relates to an electrode that includes a backing having a planar surface. The backing includes a first backing side comprising a raised section, and a second backing side comprising an indented section opposite the raised section that forms a cavity in the second backing side. Further, the backing is not electrically conductive. The electrode also includes a scrim disposed within the cavity of the indented section of the second backing side and an electrically conductive gel that is disposed at least partially within the cavity. The electrode further includes an eyelet penetrating the backing and being received in a through opening of the backing, the eyelet being electrically coupled with the electrically conductive gel. The electrode further includes a stud adapted to detachably couple the electrode to a lead wire, the stud mating with the lead wire and configured to attach to the eyelet. The scrim is a reinforcing textile material that physically reinforces the electrically conductive gel and facilitates adhesion of the electrically conductive gel to the backing.

Further disclosed herein is an exemplary method of making an electrode. The method includes providing a backing having a first backing side and a second backing side. The backing is not electrically conductive. The method also includes depositing an adhesive on the second backing side. The method further includes positioning a scrim on the adhesive. The method includes attaching a stud and an eyelet through the backing such that the scrim is proximate to the stud and the eyelet. The stud is exposed to the first backing side. The method also includes cold forming an indented section on the backing. The indented section forms a cavity therein. The cavity includes the scrim contained therein. Further, a raised section is formed opposite the cavity on the first backing side. The method further includes depositing an electrically conductive gel into the cavity such that the electrically conductive gel is fully contained within the cavity.

### Brief Description of the Drawings

Exemplary embodiments disclosed herein may be more completely understood in consideration of the following detailed description in connection with the following figures. The figures are not necessarily drawn to scale. Like numerals used in the figures refer to like components. When pluralities of similar elements are present, a single reference numeral may be assigned to each plurality of similar elements with a small letter designation referring to specific elements. When referring to the elements collectively or to a non-specific one or more of the elements, the small letter designation may be eliminated. However, it will be understood that the use of a numeral to refer to a component in a given figure is not intended to limit the component in another figure labeled with the same number.
FIG. 1 is a perspective view of an electrode according to an embodiment of the present disclosure;
FIG. 2 is a side view of the electrode of FIG. 1 according to an embodiment of the present disclosure;
FIG. 3 is an exploded view of the electrode of FIG. 1 according to an embodiment of the present disclosure;
FIG. 4 is a bottom view of the electrode of FIG. 1 according to an embodiment of the present disclosure;
FIG. 5 is a front view of a jig for forming a cavity in the electrode of FIG. 1 according to an embodiment of the present disclosure;
FIG. 5A is a front view of a portion of a first rotary drum associated with the jig of FIG. 5;
FIG. 6 is a cross-sectional view of another jig for forming the cavity in the electrode of FIG. 1 according to an embodiment of the present disclosure; and
FIG. 7 is a flowchart for a method of making the electrode according to an embodiment of the present disclosure.

### Detailed Description

In the following description, reference is made to the accompanying figures that form a part thereof and in which various embodiments are shown by way of illustration. It is to be understood that other embodiments are contemplated and may be made.

The following detailed description, therefore, is not to be taken in a limiting sense.

In the context of present disclosure, the terms "first" and "second" are used as identifiers. Therefore, such terms should not be construed as limiting of this disclosure. The terms "first" and "second" when used in conjunction with a feature or an element can be interchanged throughout the embodiments of this disclosure.

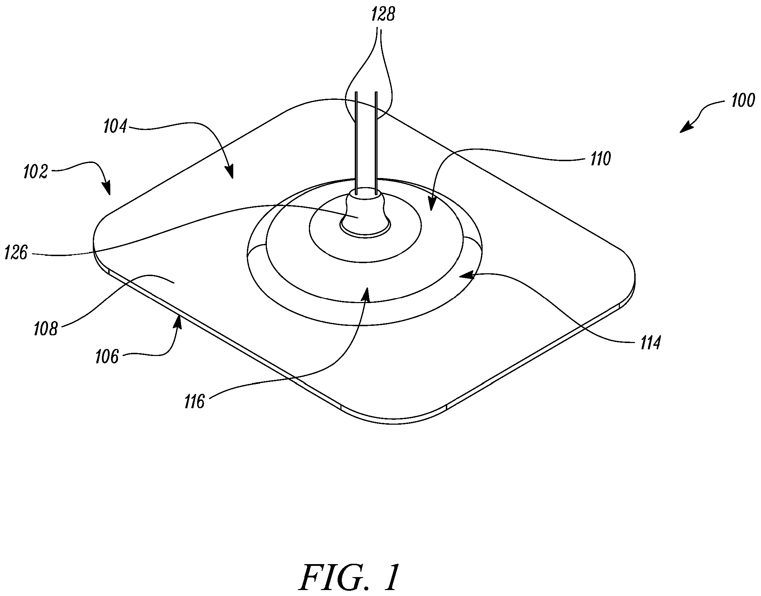

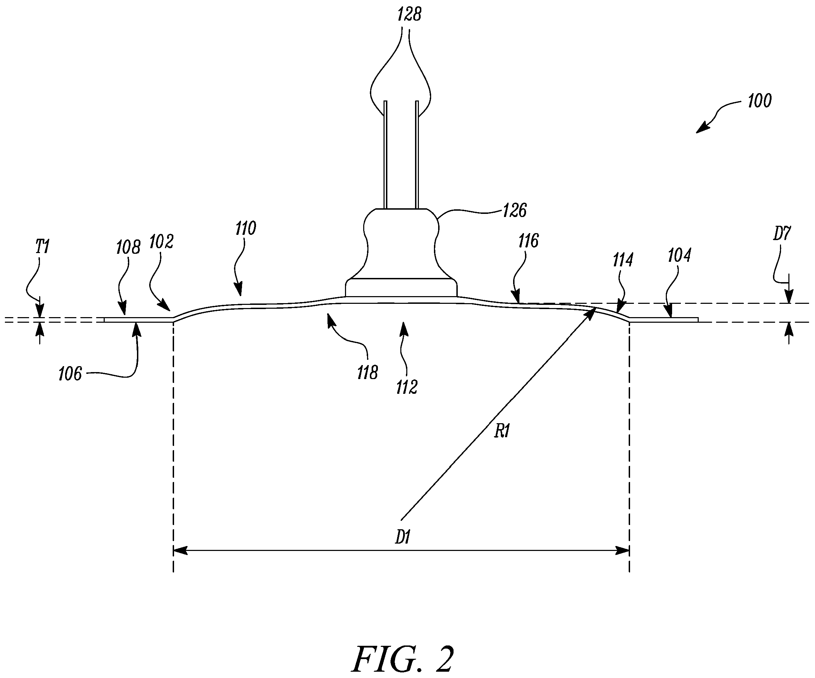

Referring to FIG. 1, a perspective view of an electrode 100 is illustrated. The electrode 100 may be embodied as an electrocardiogram electrode. In an example, the electrode 100 is radiolucent. The electrode 100 includes a backing 102. In some examples, a thickness "T1" (shown in FIG. 2) of the backing 102 is between 6 and 12 thousandths of an inch. In an example, the thickness "T1" of the backing 102 may be approximately equal to 8 millimeters (mm). The backing 102 is not electrically conductive. Further, the backing 102 has a modulus of elasticity of less than 0.9 GPa.

In an example, the backing 102 is a non-woven layer. In another example, the backing 102 is a woven layer. In yet another example, the backing 102 is a polymeric film. The polymeric film is selected from the group consisting of polyurethane, polyethylene, acrylonitrilebutadiene-styrene, polypropylene, polystyrene, polyethylene terephthalate, and combinations thereof. Further, in an example, the polymeric film includes low density polyethylene. In another example, the polymeric film includes high density polyethylene. In yet another example, the polymeric film includes at least 80 percent low density polyethylene and no greater than 20 percent high density polyethylene.

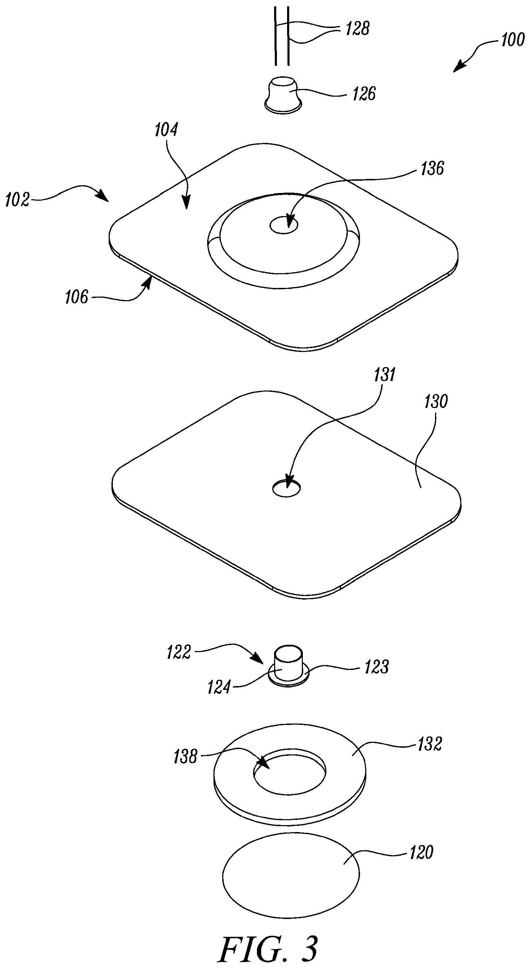

The backing 102 defines a through opening 136 (illustrated on FIG. 3) approximately at a central portion of the backing 102. The backing 102 has a first backing side 104 and a second backing side 106. The backing 102 has a planar surface 108 and a portion of the backing 102 forms a raised section 110 on the first backing side 104 and an indented section 112 on the second backing side 106 opposite the raised section 110. In the illustrated example, the raised section 110 includes a side section 114 and a planar section 116. The raised section 110can be dome-shaped. In another example, the raised section 110 may be cylindrical in shape. In some examples, the raised section 110 has a radius of curvature "R1" of 20 mm. The radius of curvature "R1" may be defined by the dome-shape of the raised section 110.

As shown in FIG. 2, the indented section 112 forms a cavity 118 therein. In one example, the cavity 118 is directly cold formed into the backing 102 through selective plastic deformation. More particularly, cold plastic deformation methods may be used for forming the cavity 118 in the backing 102 at room temperature. Cold plastic deformation may occur without supply of any additional heat. Cold plastic deformation may typically occur below a melt temperature of the material of the backing 102. In an example, the indented section 112 has a depth "D7" of no greater than 100 thousandths of an inch as measured from a plane formed by the second backing side 106 of the backing 102. Further, in some examples, the indented section 112 has an outer diameter "D1" between 15 mm and 20 mm. In one example, the indented section 112 has the outer diameter "D1" of at least 9 mm.

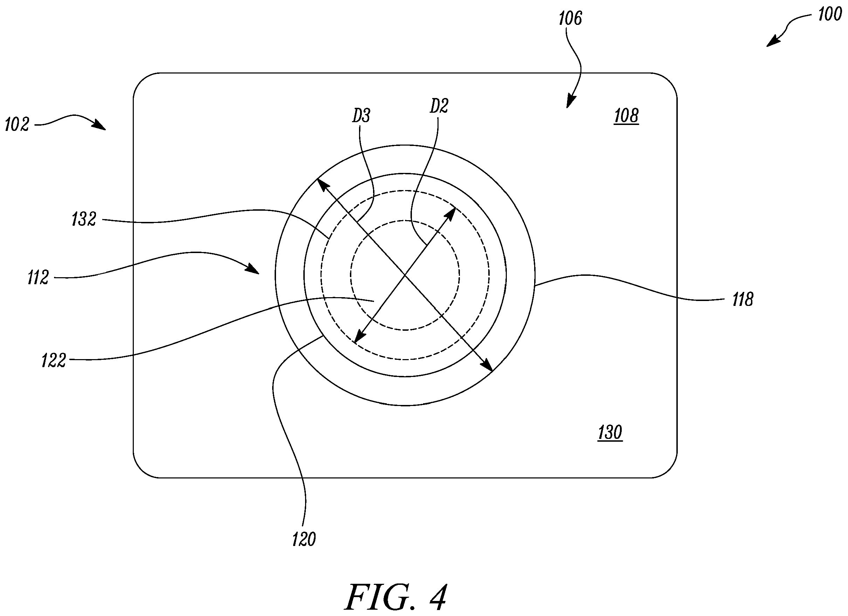

As shown in FIGS. 3 and 4, an electrically conductive gel 120 is disposed at least partially within the cavity 118 (see FIGS. 2 and 4). In an example, the electrically conductive gel 120 covers an eyelet 122 on the second backing side 106. The electrically conductive gel 120 may contain various salts, such as sodium chloride and potassium chloride, that renders the electrically conductive gel 120 ionically conductive. The electrically conductive gel 120 is disposed in the cavity 118 in a liquid form and then cured by curing means, which may be photo or thermally initiated, to convert the liquid electrically conductive gel 120 into a hydrogel.

The electrode 100 includes the eyelet 122 penetrating the backing 102 and electrically coupled with the electrically conductive gel 120. More particularly, the eyelet 122 is received in the through opening 136 of the backing 102. The eyelet 122 may embody a silver coated sensor. In various examples, the eyelet 122 may be of any suitable material, including but not limited to, silver/silver chloride plated, glass-filled ABS, stainless, or brass. The eyelet 122 has a base 123 and a post 124 (see FIG. 3) extending from the base 123. Moreover, the electrode 100 includes a stud 126 (shown in FIG. 3) adapted to detachably couple the electrode 100 to a lead wire 128 (shown in FIG. 3) and configured to attach to the eyelet 122. In an example, the electrode 100 may be connected to the lead wire 128 via an electrode connector such as a snap-style or clip-style. The eyelet 122 is coupled to the stud 126. Further, the eyelet 122 and the stud 126 may be coupled by any means known in the art. For example, the eyelet 122 may be received within an opening (not shown) of the stud 126 such that the stud 126 is connected to the eyelet 122 by a snap fit. More particularly, the post 124 of the eyelet 122 may be received within the opening of the stud 126.

In an example, the stud 126 and the eyelet 122 may be coupled to each other before forming the cavity 118. Alternatively, the stud 126 and the eyelet 122 may be coupled to each other after the cavity 118 is formed. Alternatively, the eyelet 122 and the stud 126 may be integrally formed. In some examples, the stud 126 and the eyelet 122 are made from the same material.

Further, the stud 126 is sized and configured to form an electrical connection with the lead wires 128 of a monitoring apparatus (not shown). In an example, the stud 126 may be snap fitted between the eyelet 122 and the lead wires 128 of the monitoring apparatus. It is further contemplated that the lead wires 128 of the monitoring apparatus may be coupled to the eyelet 122 by an alligator clip with attached lead wires. A design of the stud 126 may be altered such that it mates with the lead wires 128 of any particular monitoring apparatus so that the electrode 100 can be used with a number of monitoring apparatuses.

Further, the electrode 100 includes an adhesive 130. The adhesive 130 is disposed on the second backing side 106. The adhesive 130 is at least partially disposed on the second backing side 106 surrounding the indented section 112. For example, the adhesive 130 may be disposed such that the adhesive 130 includes a central through opening 131 so that the adhesive 130 surrounds the indented section 112. The adhesive 130 is a pressure-sensitive adhesive coated on the second backing side 106. The adhesive 130 may be conductive or non-conductive. In an example, the adhesive 130 includes a styrene block copolymer. Further, in some examples, the electrically conductive gel 120 is the adhesive 130. In such examples, the electrode 100 does not include a separate adhesive coated on the second backing side 106.

The electrode 100 further includes a scrim 132 disposed on a portion of the adhesive 130, proximate to the eyelet 122. The scrim 132 is a reinforcing textile material that physically reinforces the electrically conductive gel 120 and facilitates adhesion of the electrically conductive gel 120 to the backing 102. The scrim 132 has an annular shape defining a through opening 138 (see FIG. 3). In an example, the scrim 132 is formed from two sections. The scrim 132 defines an inner diameter "D2" and an outer diameter "D3". Further, the scrim 132 has a donut shape having the inner diameter "D2" to the outer diameter "D3" ratio of 1:2. In an example, the inner diameter "D2" is greater than or equal to 8 mm and the outer diameter "D3" is less than or equal to 16 mm. In some embodiments, the inner diameter "D2" of the scrim 132 may depend on a diameter of the eyelet 122. The outer diameter "D3" of the scrim 132 may be approximately equal to the outer diameter "D1" (see FIG. 2) of the indented section 112. The scrim 132 may be made of polypropylene.

For manufacturing the electrode 100, the adhesive 130 is deposited on the second backing side 106. Further, the scrim 132 is positioned at a location at which the cavity 118 needs to be formed. Moreover, the stud 126 and the eyelet 122 are attached through the backing 102 such that the scrim 132 is proximate to the stud 126 and the eyelet 122. In the illustrated example, the stud 126 and the eyelet 122 are attached before the cold forming of the indented section 112. Alternatively, the stud 126 and the eyelet 122 may be attached after the cold forming of the indented section 112.

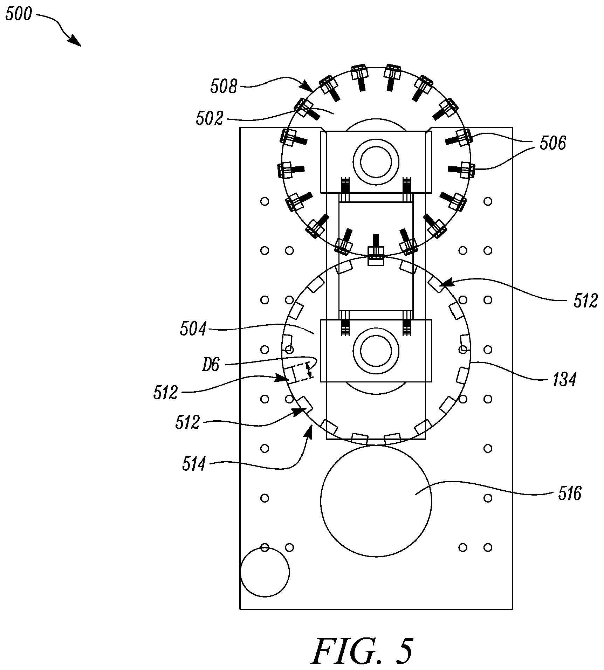

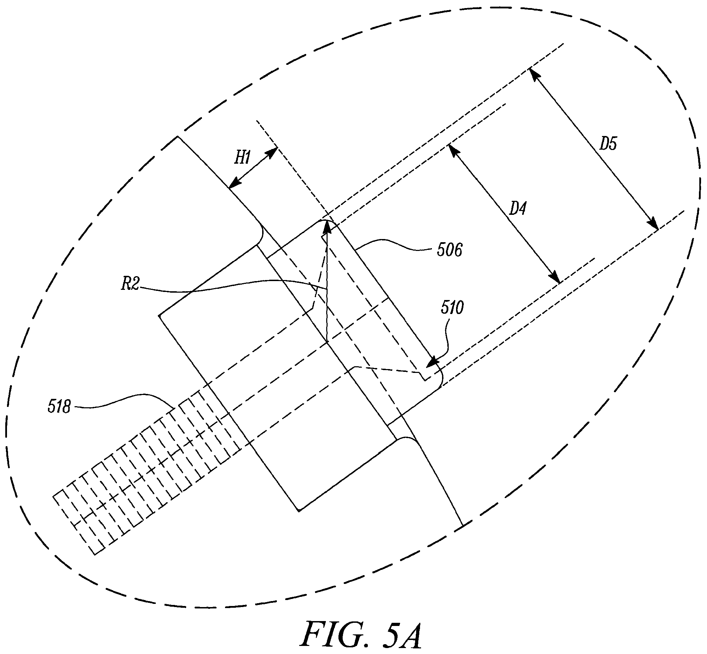

Further, as disclosed above, the indented section 112 is cold formed on the backing 102 to form the cavity 118. As shown in FIG. 5, a jig 500 is used to cold form the indented section 112. The jig 500 is centered on the stud 126 (see FIGS. 1 and 2) and the eyelet 122 (see FIG. 3) so that the cavity 118 (see FIGS. 2 and 4) is concentric with the stud 126 and the eyelet 122. Moreover, the jig 500 defines the position of the stud 126 and the eyelet 122. The cold forming of the cavity 188 includes applying force to a web 134 that includes a plurality of backings 102 using the jig 500. The jig 500 includes a first rotary drum 502 and a second rotary drum 504. In an embodiment, the second rotary drum 504 may be driven by a drive wheel 516. The second rotary drum 504 in turn drives the first rotary drum 502.

The first rotary drum 502 includes a protruding portion 506. The jig 500 illustrated herein includes a number of protruding portions 506. The protruding portions 506 are disposed on an outer surface 508 of the first rotary drum 502. In one example, the protruding portions 506 are coupled to the first rotary drum 502 via mechanical fasteners 518, such as screws. As shown in FIG. 5A, the protruding portions 506 have rounded outer edges defining an edge radius "R2" to minimize stretch and tear marks on the web 134. More particularly, the protruding portion 506 may have the edge radius "R2" from 40 to 90 thousandths of an inch. A height "H1" of the protruding portion 506 may be varied to achieve cavities 118 of desired depth. In an example, the height "H1" of the protruding portion 506 may lie between 80 mm to 180 mm. For example, the height "H1" of the protruding portion 506 may be approximately equal to 170 mm.

Further, each of the protruding portions 506 includes a recess 510 dimensioned to slide over the eyelet 122 (see FIGS. 3 and 4). More particularly, the recess 510 provides clearance to accommodate the eyelet 122 during the cold forming. Thus, the recesses 510 allow plastic deformation of the backing 102 without applying pressure on the eyelet 122. Further, the recess 510 allows usage of the mechanical fasteners 518 to couple the corresponding protruding portions 506 with the first rotary drum 502. The recess 510 has an inner diameter "D4" from 9 mm to 12 mm, inclusive, and the protruding portion 506 has an outer diameter "D5" of 11 mm to 14 mm, inclusive. The inner diameter "D4" of the recess 510 may be varied based on dimensions of the eyelet 122

Referring to FIG. 5, the second rotary drum 504 includes a recessed portion 512. The jig 500 illustrated herein includes a number of recessed portions 512. The recessed portions 512 are disposed at an outer side 514 of the second rotary drum 504. The recessed portion 512 has a diameter "D6" of 12 mm to 18 mm, inclusive. The recessed portions 512 accommodate the protruding portions 506 of the first rotary drum 502. The diameter "D6" is generally greater than the outer diameter "D5" (shown in FIG. 5A) to accommodate the protruding portion 506. In some examples, a gap measured parallel to the height "H1" may be provided between the protruding portion 506 and a lower surface defined by the recessed portion 512 so that dimensions of the cavities 118 formed on the web 134 are consistent across the web 134 and the cavities 118 includes fewer stretch or tear marks. A value of the gap may vary based on desired dimensions of the cavity 118.

Further, for forming the cavity 118, the first and second rotary drums 502, 504 rotate such that the protruding portions 506 and the recessed portions 512 move towards each other while the backing 102 is positioned between both the protruding portion 506 and the recessed portion 512. As the protruding portion 506 comes in contact with the scrim 132, the scrim 132 is pushed and a portion of the backing 102 is received within the corresponding recessed portion 512 due to force applied by the protruding portion 506 on the scrim 132, thereby forming the cavity 118.

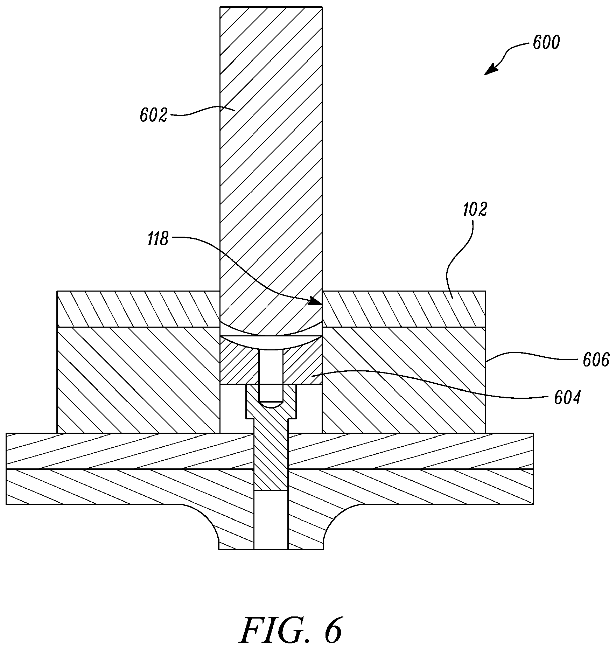

Referring now to FIG. 6, another jig 600 may be used to form the indented section 112, and more particularly, the cavity 118. The jig 600 includes a rod member 602, a fixture 606, and an insert 604 received within the fixture 606. Further, the backing 102 is placed on the fixture 606. The rod member 602 is directed towards the backing 102 such that the rod member 602 contacts the backing 102 and pushes it thereby applying a pressure on the backing 102. The pressure applied on the backing 102 causes deformation of the backing 102 to form the cavity 118. In the jig 600, the insert 604 is used to limit a length of travel of the rod member 602 to form the indented section 112 having the desired depth "D7" and diameter "D1". It should be noted that a position of the insert 604 within the fixture 606 may be varied to vary the depth "D7" and diameter "D1" of the indented section 112.

Referring to FIG. 4, the cavity 118 formed is concentric with the stud 126, the eyelet 122, and the scrim 132. After the cavity 118 is formed, the electrically conductive gel 120 is deposited into the cavity 118. Further, the electrically conductive gel 120 is cured with ultraviolet (UV) light. It should be noted that the outer diameter "D1" (see FIG. 2) of the indented section 112 that forms the cavity 118 and the depth "D7" (See FIG. 2) of the indented section 112 may be varied, based on application requirements. It should be further noted that the forming of the cavity 118 directly into the backing 102 may eliminate a need to punch a hole in the backing 102. Additionally, input material waste may also be eliminated and any need to add an additional thin covering or film to patch the hole may be eliminated. Further, forming of the cavity 118 directly into the backing 102 may also reduce an amount of the electrically conductive gel 120 that needs to be deposited in the cavity 118 and may also simplify assembly of the electrode 100 by reducing a total number of assembly steps.

The electrode 100 may embody a low-cost medical electrode that may provide a wide degree of flexibility to customize electrodes for different product lines. More particularly, dimensions of the electrode 100 may be varied as per application requirements by varying dimensions of the backing 102, a shape of the cavity 118, and plastic deformation during the fabrication method. For example, high-end electrodes may have large cavities and thus more electrically conductive gel 120 to improve performance. Additionally, the electrode 100 may use less amounts of the backing 102 and the electrically conductive gel 120. The electrode 100 may further eliminate wastage of an in-process paper release liner that is used in existing electrodes and may also eliminate the wastage of a portion of the backing that is punched out to create a gel well in existing electrodes.

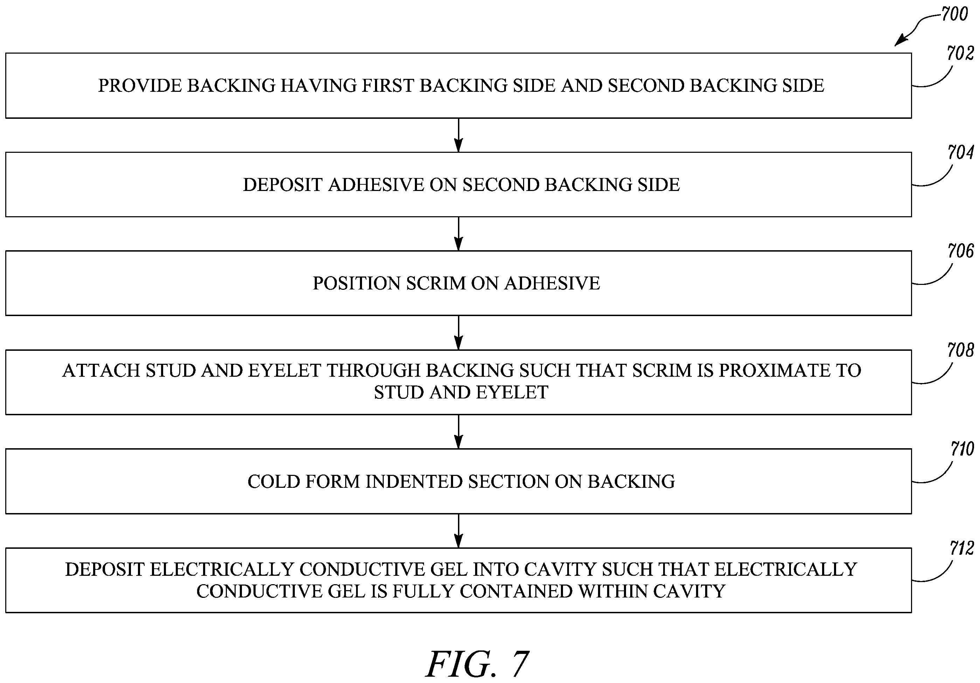

FIG. 7 is a flowchart for a method 700 of making the electrode 100. At step 702, the backing 102 having the first backing side 104 and the second backing side 106 is provided. The backing 102 is not electrically conductive. In an example, the backing 102 is a nonwoven layer. In another example, the backing 102 is a polymeric film selected from the group consisting of polyurethane, polyethylene, acrylonitrilebutadiene-styrene, polypropylene, polystyrene, polyethylene terephthalate, and combinations thereof. For example, the backing 102 is a polymeric film including at least 80 percent low density polyethylene and no greater than 20 percent high density polyethylene. The backing 102 has the thickness "T1" between 6 and 12 thousandths of an inch. Further, the backing 102 is plastically deformable.

At step 704, the adhesive 130 is deposited on the second backing side 106. At step 706, the scrim 132 is positioned on the adhesive 130. At step 708, the stud 126 and the eyelet 122 are attached through the backing 102 such that the scrim 132 is proximate the stud 126 and the eyelet 122. The stud 126 is exposed to the first backing side 104. Further, attaching the stud 126 and the eyelet 122 form a partially assembled electrode 100.

At step 710, the indented section 112 is cold formed on the backing 102. The indented section 112 forms the cavity 118 therein. The cavity 118 has the scrim 132 contained therein. Further, the raised section 110 is formed opposite the cavity 118 on the first backing side 104. The indented section 112 has the depth "D7" of no greater than 100 thousandths of an inch as measured from the plane formed by the second backing side 106. In an example, the cold forming of the indented section 112 occurs in response to attaching the stud 126 and the eyelet 122 through the backing 102. In another example, attaching the stud 126 occurs in response to cold forming the indented section 112.

Further, the cold forming includes applying force to the backing 102 with a jig 500. The jig 500 includes the protruding portion 506 and the recessed portion 512. Applying force includes moving the protruding portion 506 and the recessed portion 512 toward each other while the backing 102 is positioned between both the protruding portion 506 and the recessed portion 512. The protruding portion 506 is disposed on the first rotary drum 502. The protruding portion 506 has the edge radius "R2" from 40 to 90 thousandths of an inch.

The protruding portion 506 includes the recess 510 dimensioned to slide over the eyelet 122. The recess 510 has the inner diameter "D4" from 9 mm to 12 mm, inclusive, and the protruding portion 506 has the outer diameter "D7" of 11 mm to 14 mm, inclusive. Moreover, the recessed portion 512 is disposed on the second rotary drum 504. The recessed portion 512 has the diameter "D6" of 12 mm to 18 mm, inclusive. Further, the jig 500 is centered on the stud 126 and the eyelet 122. Moreover, the jig 500 defines the position of the stud 126 and the eyelet 122. At step 712, the electrically conductive gel 120 is deposited into the cavity 118 such that the electrically conductive gel 120 is fully contained within the cavity 118. Further, the electrically conductive gel 120 is cured with UV light.

Although specific embodiments have been illustrated and described herein, it will be appreciated by those of ordinary skill in the art that a variety of alternate and/or equivalent implementations can be substituted for the specific embodiments shown and described without departing from the scope of the present invention which is defined by the appended claims.

## Claims

1. An electrode (100) comprising:
a backing (102) having a planar surface (108), the backing (102) further comprising:
a first backing side (104) comprising a raised section (110);
a second backing side (106) comprising an indented section (112) opposite the raised section (110) that forms a cavity (118) in the second backing side (106);
wherein the backing (102) is not electrically conductive;
a scrim (132) disposed within the cavity (118) of the indented section (112) of the second backing side (106);
an electrically conductive gel (120) disposed at least partially within the cavity (118);
an eyelet (122) penetrating the backing (102) and being received in a through opening of the backing (102), the eyelet (122) being electrically coupled with the electrically conductive gel (120); and
a stud (126) adapted to detachably couple the electrode (100) to a lead wire (128), the stud (126) mating with the lead wire (128) and configured to attach to the eyelet (122),
wherein the scrim (132) is a reinforcing textile material that physically reinforces the electrically conductive gel (120) and facilitates adhesion of the electrically conductive gel to the backing (102).

2. The electrode of claim 1, wherein the backing has a modulus of elasticity of less than 0.9 GPa.

3. The electrode of claim 1, wherein the electrically conductive gel is disposed on the scrim (132) within the cavity, proximate the eyelet.

4. The electrode of claim 3, wherein the scrim is formed from two sections.

5. The electrode of claim 3 or 4, wherein the scrim has an annular shape.

6. The electrode of any of claims 1 to 5, wherein the backing (102) is a non-woven layer.

7. The electrode of any of claims 1 to 5, wherein the backing is a woven layer.

8. The electrode of any of claims 1 to 7, wherein the electrically conductive gel is at least partially disposed on the second backing (106) side surrounding the indented section.

9. The electrode of any of claims 1 to 5, wherein the backing is a polymeric film.

10. The electrode of claim 9, wherein the polymeric film comprises at least 80 percent low density polyethylene and no greater than 20 percent high density polyethylene.

11. The electrode of any of claims 1 to 10, wherein the raised section is dome-shaped.

12. The electrode of claim 11, wherein the raised section has a radius of curvature of 20 mm.

13. The electrode of any of claims 1 to 12, wherein the indented section has a depth of no greater than 2,54 mm (100 thousands of an inch as measured from a plane formed by the second backing side of the backing.

14. The electrode of any of claims 1 to 13, wherein a thickness of the backing is between 152,4 micrometers (6 thousands of an inch) and 304,8 micrometers (12 thousands of an inch).

## Patentansprüche

1. Elektrode (100), aufweisend:
eine Unterlage (102) mit einer planaren Oberfläche (108), wobei die Unterlage (102) ferner aufweist:
eine erste Unterlagenseite (104), welche einen erhöhten Abschnitt (110) aufweist;
eine zweite Unterlagenseite (106), welche einen vertieften Abschnitt (112) gegenüberliegend dem erhöhten Abschnitt (110) aufweist, der einen Hohlraum (118) in der zweiten Unterlagenseite (106) ausbildet;
wobei die Unterlage (102) nicht elektrisch leitfähig ist;
einen Gitterstoff (132), der innerhalb des Hohlraums (118) des vertieften Abschnitts (112) der zweiten Unterlagenseite (106) angeordnet ist;
ein elektrisch leitfähiges Gel (120), das zumindest teilweise innerhalb des Hohlraums (118) angeordnet ist;
eine Öse (122), welche die Unterlage (102) durchdringt und in einer Durchgangsöffnung der Unterlage (102) aufgenommen ist, wobei die Öse (122) mit dem elektrisch leitfähigen Gel (120) elektrisch gekoppelt ist; und
einen Stift (126), der angepasst ist, die Elektrode (100) lösbar mit einem Leitungsdraht (128) zu koppeln, wobei der Stift (126) mit dem Leitungsdraht (128) zusammenpasst und konfiguriert ist, an der Öse (122) angebracht zu sein,
wobei der Gitterstoff (132) ein verstärkendes Textilmaterial ist, welches das elektrisch leitfähige Gel (120) physisch verstärkt und die Haftung des elektrisch leitfähigen Gels an der Unterlage (102) unterstützt.

2. Elektrode nach Anspruch 1, wobei die Unterlage einen Elastizitätsmodul von weniger als 0,9 GPa aufweist.

3. Elektrode nach Anspruch 1, wobei das elektrisch leitfähige Gel auf dem Gitterstoff (132) innerhalb des Hohlraums nahe der Öse angeordnet ist.

4. Elektrode nach Anspruch 3, wobei der Gitterstoff aus zwei Abschnitten ausgebildet ist.

5. Elektrode nach Anspruch 3 oder 4, wobei der Gitterstoff eine ringförmige Form aufweist.

6. Elektrode nach irgendeinem der Ansprüche 1 bis 5, wobei die Unterlage (102) eine Vliesschicht ist.

7. Elektrode nach irgendeinem der Ansprüche 1 bis 5, wobei die Unterlage eine gewebte Schicht ist.

8. Elektrode nach irgendeinem der Ansprüche 1 bis 7, wobei das elektrisch leitfähige Gel zumindest teilweise auf der zweiten Unterlagenseite (106) den vertieften Abschnitt umgebend angeordnet ist.

9. Elektrode nach irgendeinem der Ansprüche 1 bis 5, wobei die Unterlage eine Polymerfolie ist.

10. Elektrode nach Anspruch 9, wobei die Polymerfolie mindestens 80 Prozent Polyethylen geringer Dichte und nicht mehr als 20 Prozent Polyethylen hoher Dichte aufweist.

11. Elektrode nach irgendeinem der Ansprüche 1 bis 10, wobei der erhöhte Abschnitt kuppelförmig ist.

12. Elektrode nach Anspruch 11, wobei der erhöhte Abschnitt einen Krümmungsradius von 20 mm aufweist.

13. Elektrode nach irgendeinem der Ansprüche 1 bis 12, wobei der vertiefte Abschnitt eine Tiefe von nicht mehr als 2,54 mm (100 Tausendstel eines Zolls) aufweist, gemessen von einer Ebene, die durch die zweite Unterlagenseite der Unterlage ausgebildet ist.

14. Elektrode nach irgendeinem der Ansprüche 1 bis 13, wobei eine Dicke der Unterlage zwischen 152,4 Mikrometer (6 Tausendstel eines Zolls) und 304,8 Mikrometer (12 Tausendstel eines Zolls) liegt.

## Revendications

1. Électrode (100), comprenant :
un support (102) ayant une surface plane (108), le support (102) comprenant en outre :
un premier côté de support (104) comprenant une section surélevée (110) ;
un second côté de support (106) comprenant une section enfoncée (112) opposée à la section surélevée (110) qui forme une cavité (118) dans le second côté de support (106) ;
dans laquelle le support (102) n'est pas conducteur d'électricité ;
une gaze (132) disposée à l'intérieur de la cavité (118) de la section enfoncée (112) du second côté de support (106) ;
un gel conducteur d'électricité (120) disposé au moins partiellement à l'intérieur de la cavité (118) ;
un œillet (122) pénétrant dans le support (102) et étant reçu dans une ouverture traversante du support (102), l'œillet (122) étant couplé électriquement au gel conducteur d'électricité (120) ; et
un goujon (126) conçu pour coupler de manière amovible l'électrode (100) à un fil conducteur (128), le goujon (126) se reliant au fil conducteur (128) et étant conçu pour se fixer à l'œillet (122),
dans laquelle la gaze (132) est un matériau textile de renforcement qui renforce physiquement le gel conducteur d'électricité (120) et facilite une adhésion du gel conducteur d'électricité au support (102).

2. Électrode selon la revendication 1, dans laquelle le support a un module d'élasticité inférieur à 0,9 GPa.

3. Électrode selon la revendication 1, dans laquelle le gel conducteur d'électricité est disposé sur la gaze (132) à l'intérieur de la cavité, à proximité de l'œillet.

4. Électrode selon la revendication 3, dans laquelle la gaze est formée de deux sections.

5. Électrode selon la revendication 3 ou 4, dans laquelle la gaze a une forme annulaire.

6. Électrode selon l'une quelconque des revendications 1 à 5, dans laquelle le support (102) est une couche non tissée.

7. Électrode selon l'une quelconque des revendications 1 à 5, dans laquelle le support est une couche tissée.

8. Électrode selon l'une quelconque des revendications 1 à 7, dans laquelle le gel conducteur d'électricité est au moins partiellement disposé sur le second côté de support (106) entourant la section enfoncée.

9. Électrode selon l'une quelconque des revendications 1 à 5, dans laquelle le support est un film polymère.

10. Électrode selon la revendication 9, dans laquelle le film polymère comprend au moins 80 pour cent de polyéthylène basse densité et pas plus de 20 pour cent de polyéthylène haute densité.

11. Électrode selon l'une quelconque des revendications 1 à 10, dans laquelle la section surélevée est en forme de dôme.

12. Électrode selon la revendication 11, dans laquelle la section surélevée a un rayon de courbure de 20 mm.

13. Électrode selon l'une quelconque des revendications 1 à 12, dans laquelle la section enfoncée a une profondeur de pas plus de 2,54 mm (100 millièmes de pouce) telle que mesurée à partir d'un plan formé par le second côté de support du support.

14. Électrode selon l'une quelconque des revendications 1 à 13, dans laquelle une épaisseur du support est comprise entre 152,4 micromètres (6 millièmes de pouce) et 304,8 micromètres (12 millièmes de pouce).
